# EUROPEAN PATENT SPECIFICATION

(11) **EP 1 221 605 B1**
(45) Date of publication and mention of the grant of the patent: **10.12.2003**
(21) Application number: 01100435.5
(22) Date of filing: 08.01.2001
(51) Int. Cl.: G01N 1/08, G01N 1/14

(54) **Portable sampling lance**
Tragbare Proben-Entnahmelanze
Lance portable de prélèvement d'échantillons

(43) Date of publication of application: 10.07.2002
(73) Proprietor: EUROPEAN COMMUNITY, 1049 Brussels (BE)
(72) Inventor: Srtoka, Joerg, 47137 Duisburg (DE)
(74) Representative: Kihn, Pierre Emile Joseph

(56) References cited:
- EP-A- 0 411 932
- FR-A- 2 161 170
- FR-A- 2 628 207
- GB-A- 1 399 990
- US-A- 2 904 443

## Description

The present invention generally relates to a portable sampling lance for rapid and non-destructive sampling of unpacked bulk food and the like. Such sampling lences are described for example in EP-0 411 932, FR-2 628 207, US-2 904 443, FR-2 161 170 and GB-1 399 990.

The sampling of large and unpacked food shipments is normally performed according to legislative set sampling plans. Since the contaminants are usually not evenly distributed in the shipment, the distribution of the contaminant is a crucial parameter that is taken into account by sampling several subsamples and analysing them with the intention to obtain a realistic average contamination with a certain statistical security.

However, the sampling and the subsampling are labour intensive and not very practical.

An alternative to the above is the sampling of bulk size food loads. In US 5,942,669, bulk products or palletised products are loaded en masse into an airtight chamber and subjected to physical agitation, f.ex. vibration, blasts of pressurised air into the chamber, jets of pressurised air directed at the products, and cycles of pressurisation and depressurisation, to release both vapours and particulates from both the interiors and the outer surfaces of the cargo items. The object of this physical agitation is to knock loose aerosol-size contaminant particles on the exterior and interior surfaces of the items, and to encourage the vaporisation of low vapour pressure chemical residues into the air in the chamber. In this manner, contaminant particulates from the outer surfaces of the items and from the interiors of the items are placed in suspension in the air in the chamber, and contaminant vapours from the outer surfaces of the items and from the interiors of the items are mixed with the air in the chamber. During the depressurisation phases, air withdrawn from the chamber is passed through a collection system to collect the vapours and particulates. The collected vapours and particulates then are transferred to conventional analytic instruments for identification. If a targeted substance such as a pesticide residue, a trace of an explosive, or a trace of an illegal drug, is identified during this analysis, appropriate steps may be taken, including generating an audible and visible alarm.

A problem with this type of sampling is that the apparatus is not transportable and the samples have to be taken to the apparatus.

### Object of the invention

The object of the present invention is to provide a portable system for sampling large, unpacked shipments of foods and the like.

### General description of the invention

In order to overcome the above mentioned problem, the present invention provides a portable sampling lance for rapid and non-destructive sampling of unpacked bulk food and the like comprising:
a head portion with a chamber, said chamber having an intake opening,
a duct connected to said head portion,
a collecting device connected to said duct,
a suction device connected to said duct, characterized in that:
   the intake opening of the chamber of the head portion is covered by a grid, and
wherein said head portion has a front end and a rear end and wherein the intake opening of the chamber is oriented towards the rear end of the head portion.

The portable lance is dug into a heap of bulk food such as corn, rice etc. Since the opening of the chamber is oriented towards the rear end of the head portion and since the opening is covered by a grid, only small particles like dust and rub off material are admitted into the chamber whereas the larger particles like the corn or rice grains are retained outside said chamber. The dust and rub off material particles are sucked form the chamber through the duct and are collected by the collecting device.

The fact that the opening of the chamber is turned the opposite way as the movement used to dig or push the lance into the mass of product to be analysed, protects the grid form mechanical constraints. Furthermore, the grid does not easily become clogged during the introduction of the portable lance into the product to be sampled.

The mesh of the grid is adapted to the product to be sampled and is chosen so that the particles or individual grains of the food are retained outside the chamber. The grid has preferably a mesh size of 0.2 mm to 3 mm depending on the type and the hardness of material to be analysed.

The head portion may be substantially spearhead or hook shaped.

According to a preferred embodiment, the head portion further comprises vibrating means. These vibrating means allow the portable lance to be introduced into the product more easily. Furthermore, they enhance the quantity of rub-off and dust collected in a certain periods of time.

Preferably, the collection device is a filter means such as a round filter made of glass fibre, cellulose, nylon etc or an electrostatic device

According to another preferred embodiment, the chamber has a substantially conical shape, the part comprising the intake opening being larger in diameter than the part connected to the suction duct so the dust and rub off material are accelerated once they penetrate into the chamber.

In order to further enhance the uptake of dust and rub off material, the grid may be charged electrically. In that case the grid must be insulated from the chamber and the counter-electrode can be placed inside the chamber.

### Detailed description with respect to the figures

The present invention will be more apparent from the following description of not limiting embodiments with reference to the attached drawings, wherein
Fig.1: shows a schematic representation of a first embodiment of the head portion of the portable lance;
Fig.2: shows a schematic representation of a second embodiment of the head portion of the portable lance;
Fig.3: shows a schematic representation of an embodiment of a collecting device of the portable lance;

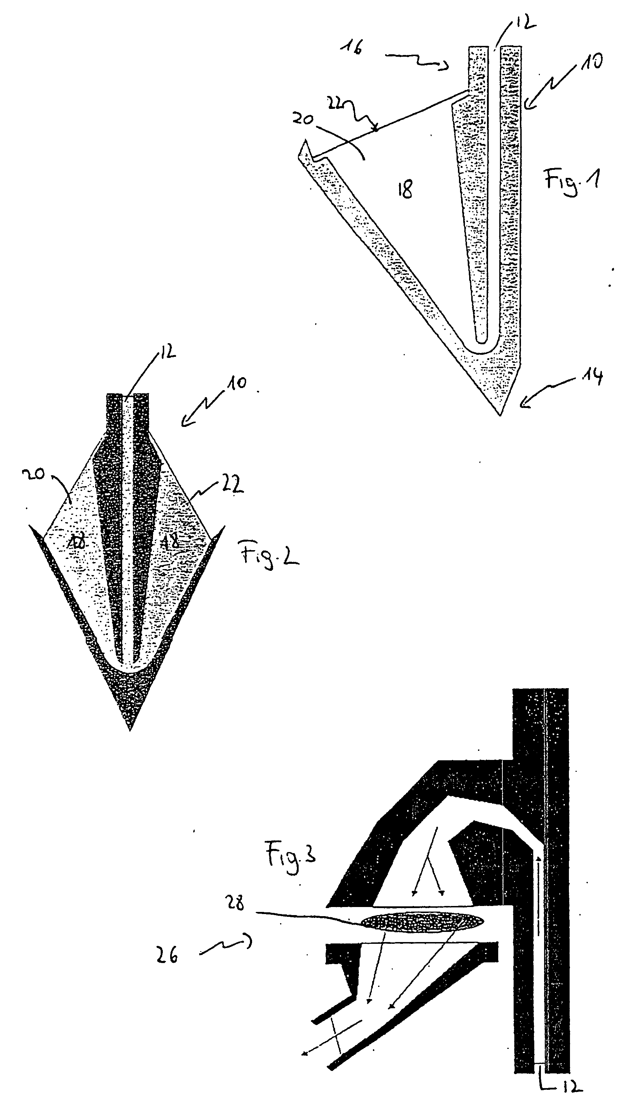

Referring now to the drawing, reference numeral 10 in FIGS. 1 and 2 identifies the head portion of a portable lance.

The portable sampling lance further comprises a duct 12 connecting the head portion to a collecting device. The portable lance is connected to a suction device supplying a reduced pressure to the head portion 10 and the chamber 14 of the lance.

The head portion 10 has a front end 14 and a rear end 16 and a chamber 18 located inside the head portion. The intake opening 20 of the chamber 18 is oriented towards the rear end 16 of the head portion 10 i.e. the intake opening 20 faces rearwardly when the lance is introduced into the sample to be analysed. The intake opening 20 is covered by a grid 22 so that only grains, which are smaller than the perforations of the gird, pass through the grid into the chamber 18. The grains, which are larger in diameter than the perforations in the grid 22, will remain outside of the chamber 18. Since the intake opening 20 is turned rearwardly, the grid 22 will not become easily clogged by grains of a grain size lager than the perforations in the grid. Furthermore, the grid 22 is submitted to less mechanical constraints during the introduction of the lance into the material to be analysed.

Once the head portion 10 of the lance is introduced into a heap of bulk material, the dust and rub off material is sucked into the intake opening 20 of the head portion 10, by virtue of the reduced pressure produced therein by a suction blower or the like (not shown). The dust and rub off material, which is sucked into the intake opening 20 of the head portion 10, passes through the grid 22 into the chamber 18, the interior of which is defined by a conical wall structure. At its bottom, the chamber is provided with an opening 24 connecting the chamber 18 to the duct 12.

As indicated above, the fact that the intake opening 20 faces generally backwardly, i.e. towards the rear end 16 of the head portion 10 means that no material can pass into the chamber18, without having been drawn thereinto by a suction effect. In that respect, by suitably adjusting the reduced pressure generated by the suction device and/or by virtue of a suitable choice of the cross-section of the suction intake opening of the duct 12, it is possible to define the range of grain sizes which is to be drawn into the duct through the intake opening 20 of the chamber 18.

The head portion 10 of the lance which may be for example from 100 mm to 300 mm in diameter and have a length of 100 mm to 400 mm, is connected by way of suitable conduits to a means for producing a reduced pressure as indicated in the form of a suction blower, which is operable to produce a reduced pressure that is applied to the intake opening of the chamber.

The above-described structure and mode of operation ensure that the fine-grain material which is sucked into the chamber 18 is representative of the portion of the bulk material from which the sucked-in material is drawn.

Mounted to the head portion 10 of the portable lance is a vibrator, which is not illustrated, in order not to encumber the drawing. The configuration and arrangement of the vibrator and the appropriate connections thereto will be familiar to any man skilled in the art. The vibrator makes it easier to introduce the lance into the bulk material to be analysed. Furthermore, grains being too large to pass the grid 22 on the intake opening can be removed by actuating the vibrator at certain intervals of time. The resulting vibrational effect causes at least a large part of the grains clinging to the outside surface of the grid 22 to drop off. It is also possible for the vibrator to be operated in a continuous mode of operation in order to assist with the passage of the dust and rub off material through the grid of the intake opening and also through the layer of grains which is possibly clinging to the outside of the latter. In that mode of operation however, the vibrator would only produce a light vibration so that the material which is sucked into the arrangement by the suction force produced by the suction device is held to the outside surface of the grid and the vibration effect accelerates the passage of the dust and rub off material through the grid.

The dust and rub off material and other fine-grain material, which is drawn into the intake opening 20, first passes into the chamber 18 and then through the duct 12 into to the collecting device 26 under the effect of the reduced pressure generated by the suction device. The fine grain material is then collected by the collecting device 26, which is f. ex. a filter 28 or an electrostatic device. The time at which the filter 28 is to be removed for analysis can be established by measuring the reduced pressure downstream of the collecting device, in the direction of flow of the air through the arrangement. A rise in the reduced pressure at the downstream location as mentioned indicates that the filter 28 is clogged to a greater or lesser degree.

The collecting device 26 may also be in the form of a cyclone separator is connected into the circuit between the head portion 10 of the lance and the suction device.

The collected fine grain material is analysed according to conventional analytical methods.

It will be seen from the foregoing that the method and apparatus for taking samples from a bulk material may be applied to any suitable material such as corn, barley, wheat, rice, rye, coffee beans, soy beans, nuts (with and without shells), dried chilli peppers or other 'non ground' food products, for subsequently determining respective chemical contaminants contained in the sampled material.

It will be further appreciated that the above-described method and apparatus according to the invention have been described solely by way of example and illustration thereof and that various modifications and alterations may be made therein without thereby departing from the scope of the invention as claimed.

### Reference list

- 10: head portion
- 12: duct
- 14: front end
- 16: rear end
- 18: chamber
- 20: intake opening
- 22: grid
- 24: opening
- 26: collecting device
- 28: filter

## Claims

1. Portable sampling lance for rapid and non-destructive sampling of unpacked bulk food comprising:
a head portion (10) with a chamber (18), said chamber having an intake opening (20),
a duct (12) connected to said head portion (10),
a collecting device (26) connected to said duct (12),
a suction device connected to said duct (12),
**characterised in that**
the intake opening (20) of the chamber (18) of the head portion (10) is covered by a grid (22), and
wherein said head portion (10) has a front end (14) and a rear end (16) and wherein the intake opening (20) of the chamber (18) is oriented towards the rear end (16) of the head portion (10).

2. Sampling lance according to claim 1, the grid (22) has a mesh size of between 0.2 mm and 3 mm.

3. Sampling lance according to claim 1 or 2, wherein the head portion (10) further comprising a vibrating means.

4. Sampling lance according to any one of the preceding claims, wherein the head portion (10) is substantially spearhead shaped.

5. Sampling lance according to any one of the preceding claims, wherein the head portion (10) is substantially hook shaped.

6. Sampling lance according to any one of the preceding claims, wherein the chamber (18) has a substantially conical shape.

7. Sampling lance according to any one of the preceding claims, wherein the collection device is a filter means (28).

8. Sampling lance according to any one of the claims 1 to 3, wherein the collection device (26) is an electrostatic device.

9. Sampling lance according to any one of the preceding claims, wherein the grid (22) is charged electrically.

## Patentansprüche

1. Tragbare Proben-Entnahmelanze zur schnellen und zerstörungsfreien Probenentnahme bei unverpackten Massengut-Nahrungsmitteln, umfassend:
einen Kopfteil (10) mit einer Kammer (18), wobei die Kammer eine Einlassöffnung (20) hat;
einen Kanal (12), der mit dem Kopfteil (10) verbunden ist;
eine Sammelvorrichtung (26), die mit dem Kanal (12) verbunden ist;
eine Saugvorrichtung, die mit dem Kanal (12) verbunden ist;
**dadurch gekennzeichnet, dass**
die Einlassöffnung (20) der Kammer (18) des Kopfteils (10) durch ein Gitter (22) abgedeckt ist, und
wobei der Kopfteil (10) ein vorderes Ende (14) und ein hinteres Ende (16) hat und wobei die Einlassöffnung (20) der Kammer (18) auf das hintere Ende (16) des Kopfteils (10) gerichtet ist.

2. Proben-Entnahmelanze nach Anspruch 1, wobei das Gitter (22) eine Maschenweite zwischen 0,2 mm und 3 mm aufweist.

3. Proben-Entnahmelanze nach Anspruch 1 oder 2, wobei der Kopfteil (10) ferner ein Rüttelmittel umfasst.

4. Proben-Entnahmelanze nach irgendeinem der vorangehenden Ansprüche, wobei der Kopfteil (10) im Wesentlichen speerspitzenförmig ist.

5. Proben-Entnahmelanze nach irgendeinem der vorangehenden Ansprüche, wobei der Kopfteil (10) im Wesentlichen hakenförmig ist.

6. Proben-Entnahmelanze nach irgendeinem der vorangehenden Ansprüche, wobei die Kammer (18) im Wesentlichen kegelförmig ist.

7. Proben-Entnahmelanze nach irgendeinem der vorangehenden Ansprüche, wobei die Sammelvorrichtung ein Filtermittel (28) ist.

8. Proben-Entnahmelanze nach irgendeinem der Ansprüche 1 bis 3, wobei die Sammelvorrichtung (26) eine elektrostatische Vorrichtung ist.

9. Proben-Entnahmelanze nach irgendeinem der vorangehenden Ansprüche, wobei das Gitter (22) elektrisch aufgeladen ist.

## Revendications

1. Lance portable d'échantillonnage pour l'échantillonnage rapide et non destructif d'aliments en vrac, non-emballés, comprenant :
une portion de tête (10) avec une chambre (18), ladite chambre présentant une ouverture d'admission (20),
une conduite (12) reliée à ladite portion de tête (10),
un dispositif collecteur (26) relié à ladite conduite (12),
un dispositif d'aspiration relié à ladite conduite (12),
**caractérisée en ce que**
l'ouverture d'admission (20) de la chambre (18) de la portion de tête (10) est recouverte par une grille (22), et
dans laquelle ladite portion de tête (10) présente une extrémité avant (14) et une extrémité arrière (16) et dans laquelle l'ouverture d'admission (20) de la chambre (18) est orientée vers l'extrémité arrière (16) de la portion de tête (10).

2. Lance d'échantillonnage selon la revendication 1, dans laquelle la grille (22) possède une taille d'ouverture de maille comprise entre 0,2 mm et 3 mm.

3. Lance d'échantillonnage selon la revendication 1 ou 2, dans laquelle la portion de tête (10) comprend de plus un moyen vibrant.

4. Lance d'échantillonnage selon l'une quelconque des revendications précédentes, dans laquelle la portion de tête (10) est essentiellement en forme de tête de lance.

5. Lance d'échantillonnage selon l'une quelconque des revendications précédentes, dans laquelle la portion de tête (10) est essentiellement en forme de crochet.

6. Lance d'échantillonnage selon l'une quelconque des revendications précédentes, dans laquelle la chambre (18) présente une forme essentiellement conique.

7. Lance d'échantillonnage selon l'une quelconque des revendications précédentes, dans laquelle le dispositif collecteur est un moyen de filtre (28).

8. Lance d'échantillonnage selon l'une quelconque des revendications 1 à 3, dans laquelle le dispositif collecteur (26) est un dispositif électrostatique.

9. Lance d'échantillonnage selon l'une quelconque des revendications précédentes, dans laquelle la grille (22) est chargée électriquement.
